# EUROPÄISCHE PATENTSCHRIFT

(11) **EP 1 083 834 B1**
(45) Veröffentlichungstag und Bekanntmachung des Hinweises auf die Patenterteilung: **20.09.2006**
(21) Anmeldenummer: 00922622.6
(22) Anmeldetag: 10.04.2000
(51) Int. Cl.: A61B 17/34, A61M 25/02

(54) **VORRICHTUNG ZUM SCHAFFEN EINES TRANSKUTANEN ZUGANGES ZU EINEM KÖRPERINNEREN HOHLORGAN**
DEVICE FOR PRODUCING A TRANSCUTANEOUS ACCESS TO A HOLLOW VISCUS IN THE INTERIOR OF THE BODY
DISPOSITIF PERMETTANT DE PRATIQUER UN ACCES TRANSCUTANE A UN ORGANE INTERNE CREUX

(30) Priorität: 09.04.1999 DE 19916088
(43) Veröffentlichungstag der Anmeldung: 21.03.2001
(73) Patentinhaber: Karl Storz GmbH & Co. KG, 78532 Tuttlingen (DE)
(72) Erfinder: KURFESS, Karlheinz, D-55469 Simmern (DE); SALVERMOSER, Markus, D-78532 Tuttlingen (DE)
(74) Vertreter: Weller, Wolfgang
(86) Internationale Anmeldenummer: PCT/EP2000/003170
(87) Internationale Veröffentlichungsnummer: WO 2000/061016

(56) Entgegenhaltungen:
- DE-A- 19 543 011
- US-A- 4 069 826
- US-A- 5 073 166
- US-A- 5 312 351
- US-A- 5 549 657
- US-A- 5 792 112
- US-A- 5 855 569

## Beschreibung

Die Erfindung betrifft eine Vorrichtung zum Schaffen eines transkutanen Zuganges zu einem körperinneren Hohlorgan, insbesondere zum Magen, mit einer hohlrohrförmigen flexiblen Sonde, die transkutan in das Hohlorgan legbar ist, mit einer starren Hülse, die auf einen vom Körper abstehenden Abschnitt der Sonde derart aufbringbar ist, daß die Hülse diesen Abschnitt umgreift.

Eine derartige Vorrichtung ist aus der DE 195 43 011 C2 bekannt.

Bei dieser bekannten Vorrichtung wird eine schlauchartige flexible Sonde in den Magen eingeführt und durch die Magenwand und die Bauchdecke zur Außenseite hin geführt. Eine Rückhaltescheibe am anderen Ende der Sonde verhindert ein Herausgleiten der Sonde aus dem Magen. Über den vom Körper abstehenden Abschnitt der Sonde wird eine Versteifungshülse aufgeschoben, und zwar über dessen Außenseite. Dies soll dabei so geschehen, daß möglichst keine Relativverschiebung zwischen Sonde und aufgeschobener Versteifungshülse stattfindet, was, ohne daß dies in diesem Dokument ausdrücklich erwähnt ist, wohl durch Reibschluß erfolgt .

Der Zusammenbau aus vom Körper abstehendem Abschnitt der Sonde und übergeschobener Versteifungshülse dient dazu, einen transkutanen Zugang zu dem körperinneren Hohlorgan, hier dem Magen, zu schaffen. Durch diesen Zugang können beispielsweise Instrumente in den Körper eingeführt werden. Um ein Entweichen von Darm- und Mageninhalt oder von Insufflationsgas aus dem Hohlorgan zu verhindern, wird am außenseitigen Ende der Versteifungshülse ein Ventilelement aufgesetzt.

Nachteilig an dieser Vorrichtung ist, daß Relativbewegungen zwischen dem vom Körper abstehenden Abschnitt der Sonde und der aufgeschobenen Versteifungshülse nicht ausgeschlossen werden können.

Selbst wenn man einen relativ festen Reibschluß zwischen der Außenseite der flexiblen Sonde und der Innenseite der auf diese aufgeschobenen Versteifungshülse vorsieht, kann nicht ausgeschlossen werden, daß bei ruckartigen Bewegungen, z.B. beim Husten, dennoch eine Relativverschiebung stattfindet, was unerwünscht ist.

Dieser feste Reibschluß beeinträchtigt auch sehr die Handhabung beim Anlegen der Versteifungshülse auf die Sonde. Beim Anlegen muß ja die Versteifungshülse über den vom Körper abstehenden Abschnitt geschoben werden. Bei starkem Reibschluß ist dies nur schwer und mit ruckartigen Bewegungen durchzuführen, so daß es auch schwierig ist, einen Sitz nachträglich zu korrigieren.

Es ist daher Aufgabe der vorliegenden Erfindung, hier Abhilfe zu schaffen durch eine Vorrichtung, über die ein transkutaner Zugang einfach und für den Patienten möglichst atraumatisch bewerkstelligt werden kann, um insbesondere einen sicherer Sitz der an der Sonde angebrachten Hülse sicherzustellen.

Erfindungsgemäß wird die Aufgabe dadurch gelöst, daß eine Klemmvorrichtung zum lösbaren, jedoch festen Verbinden der Sonde mit der Hülse vorhanden ist, die bewegliche Klemmelemente aufweist, welche zwischen einer nicht-klemmenden und einer klemmenden Stellung bewegbar sind.

Dadurch, daß die beweglichen Klemmelemente in eine nicht-klemmende Stellung bewegbar sind, kann die Hülse ohne Widerstand auf den vom Körper abstehenden Abschnitt der Hülse aufgeschoben und richtig positioniert werden. Ein zutreffendes Positionieren wird dadurch bewerkstelligt, daß an der Sonde etwas gezogen wird, so daß deren innere Scheibe die Magenwand an die Bauchdecke drückt. Nach Einstellen der zutreffenden gewünschten Relativposition zwischen Sonde und aufgeschobener Hülse wird die Klemmvorrichtung betätigt und die beweglichen Klemmelemente in die klemmende Stellung gebracht. Die Klemmwirkung kann nun so ausgewählt werden, daß auch bei ruckartigen Bewegungen des Patienten, beispielsweise beim Husten, keine Relativverschiebung stattfindet.

Über die Klemmvorrichtung ist eine lösbare, jedoch feste Verbindung zwischen der äußeren starren Hülse und dem darin ausgerichteten Abschnitt der Sonde möglich, so daß keine Relativbewegungen zwischen diesen beiden Bauteilen im geklemmten Zustand möglich sind. Die Sonde ist lediglich auf die Länge der Vorrichtung abzulängen, so daß dann, wie das die Operateure bei Trokaren gewohnt sind, die Instrumente über ein hülsenartiges Bauteil eingeführt werden können. Nach Abziehen der Vorrichtung von der Sonde steht noch ein ausreichend langer Abschnitt vom Körper ab, über den dann zur intragastralen Langzeiternährung wieder entsprechende Anschlüsse angebracht werden können oder auch anderweitige Abschlußelemente. Auf diesen Abschnitt kann dann, falls es notwendig ist, erneut die Vorrichtung aufgebracht werden.

Dadurch, daß die Hülse die Sonde passend umgreift und führt, ist diese derart exakt ausgerichtet, daß sie völlig konzentrisch in der starren Hülse sitzt. Dadurch ist auch die proximale stirnseitige Endfläche der Sonde exakt ringförmig ausgerichtet, so daß keine Gefahr besteht, daß beim Einführen von Instrumenten diese gegen diese Stirnkante stoßen, was der Fall sein könnte, wenn diese beispielsweise oval oder in sonstiger Weise verformt wäre. Dies ist ein wesentlicher Beitrag zur Betriebssicherheit der Vorrichtung.

In einer weiteren Ausgestaltung der Erfindung ist ein Fußteil zum Aufsetzen der Vorrichtung auf der Körperfläche vorgesehen.

Diese Maßnahme hat den Vorteil, daß ein exakt ausgerichteter Sitz der Vorrichtung auf der Körperfläche gewährleistet ist.

In einer weiteren Ausgestaltung ist eine Ventilanordnung vorgesehen, durch die der von der Sonde geschaffene Zugang dicht verschließbar ist.

Diese Maßnahme hat den Vorteil, daß die Vorrichtung selbst schon diese Ventilanordnung aufweist, so daß keine umständlichen Anschlüsse an der Sonde selbst bewerkstelligt werden müssen, sondern die Vorrichtung lediglich auf die Sonde aufgeschoben bzw. aufgesetzt werden muß.

In einer weiteren Ausgestaltung der Erfindung weist die Klemmvorrichtung bewegliche Klemmelemente auf, die an die Außenseite der Sonde anlegbar sind.

Diese Maßnahme hat den Vorteil, daß nach Aufschieben der Vorrichtung auf die Sonde die Klemmelemente an die Außenseite der Sonde anlegbar sind, und dadurch ein Verklemmen zwischen der Vorrichtung und der Sonde bewerkstelligt werden kann.

In einer weiteren Ausgestaltung der Erfindung sind die beweglichen Klemmelemente flächig an die Außenseite der Sonde anlegbar.

Diese Maßnahme hat den Vorteil, daß durch eine großflächige Anlage schon durch einen relativ geringen Anpreßdruck ein solcher Reibschluß geschaffen wird, daß die Vorrichtung axial unverrückbar an der Sonde angeklemmt ist. Dies bildet eine besonders schonende Anklemmvorrichtung, ohne die Materialstruktur, die Form oder die Außenseite der Sonde nachteilig zu beeinträchtigen. Dieser Reibschluß kann dann durch entsprechende Oberflächenausgestaltung bzw. Rauheit der Klemmelemente weiter verstärkt werden.

In einer weiteren Ausgestaltung der Erfindung weisen die Klemmelemente Vorsprünge auf, die beim Klemmschluß in die flexible Sonde eindringen, ohne jedoch dabei das Lumen der Sonde zu verändern.

Diese Maßnahme hat den Vorteil, daß durch die Vorsprünge besonders sichergestellt ist, daß auch bei ungeschickter Handhabung oder versehentlichem Verhaken der Vorrichtung mit anderen Instrumenten kein Lösen der Vorrichtung von der Sonde stattfindet. Durch entsprechend flexibles Material ist es möglich, daß die Vorsprünge von außen in die Sonde eindringen, ohne dabei das Lumen der Sonde zu verändern, so daß auch im geklemmten Zustand der volle Innenquerschnitt als Instrumentenkanal zur Verfügung steht.

In einer weiteren Ausgestaltung der Erfindung ist das Fußteil als separates Bauteil ausgebildet.

Diese Maßnahme hat den Vorteil, daß das Anlegen der Vorrichtung dahingehend erleichtert ist, daß zunächst einmal das Fußteil über die vom Körper abstehende Sonde geschoben wird und das Fußteil dann am Körper an einer geeigneten Ausrichtung und Stelle positioniert wird.

In einer weiteren Ausgestaltung der Erfindung weist das Fußteil die Klemmvorrichtung auf.

Diese Maßnahme hat den Vorteil, insbesondere mit der zuvor genannten Maßnahme, daß das Fußteil nach dessen Ausrichtung mit der Sonde verklemmt werden kann. Dabei können auch noch Korrekturen durchgeführt werden, die wesentlich einfacher für den Operateur durchzuführen sind, als wenn schon die komplette Vorrichtung angebracht wäre.

In einer weiteren Ausgestaltung der Erfindung weist die Klemmvorrichtung zwei etwa halbschalenförmige Klemmelemente auf.

Diese Maßnahme hat den Vorteil, daß diese Klemmelemente raumsparend angeordnet werden können, über einen großen Flächenbereich die Sonde umgreifen können und diese dann ebenfalls zusätzlich ausrichtend, dichtend und stützend im Klemmschluß umgreifen.

In einer weiteren Ausgestaltung der Erfindung weist die Klemmvorrichtung eine über einen äußeren Hebel verdrehbare Exzenterwelle auf, deren Exzenter mit zumindest einem der beweglichen Klemmelemente in Wirkverbindung steht.

Diese Maßnahme hat den handhabungstechnischen Vorteil, daß die Vorrichtung zunächst bei offener Klemmvorrichtung aufgeschoben werden kann, wobei die Hebelstellung dem Operateur diese Stellung eindeutig anzeigt. Nach einer zutreffenden Ausrichtung braucht lediglich der Hebel umgelegt werden, um den Verklemmvorgang mit der Sonde einfach durchzuführen. Die dann umgelegte Stellung zeigt dem Operateur eindeutig diese Position an.

In einer weiteren Ausgestaltung der Erfindung weist das Fußteil eine durchgehende, kanalartige Öffnung auf, in der radial beweglich die Klemmelemente angeordnet sind.

Diese Maßnahme hat den Vorteil, daß die Klemmelemente in raumsparender Weise in dem Fußteil integriert sind, in geöffnetem Zustand ein Aufschieben bzw. Einfädeln der Sonde einfach ermöglichen und dann durch eine radiale Bewegung in die Öffnung hinein die Sonde festklemmen.

In einer weiteren Ausgestaltung der Erfindung weist die Klemmvorrichtung ein Schlauchstück auf, durch das die Sonde hindurchgeführt ist, und die beweglichen Klemmelemente der Klemmvorrichtung klemmen das Schlauchstück, wodurch die im Schlauchstück aufgenommene Sonde mit diesem verklemmt ist.

Diese Maßnahme hat den Vorteil, daß die Klemmelemente nicht unmittelbar mit der Sonde in Eingriff stehen sondern mit einem Schlauchstück, durch das die Sonde hindurchgeführt ist. Dadurch, daß das gesamte Schlauchstück großflächig auf die Sonde gepreßt wird, kann besonders schonend über den großflächigen Sitz die ausreichende Klemmkraft ausgeübt werden, ohne die Sonde zu beeinträchtigen. Durch entsprechende Oberflächenausgestaltung der Schlauchmaterialien oder schon allein durch die Auswahl der Materialien selbst ist schon eine gewisse Klemmwirkung zu erzielen, die eine Relativbewegung zwischen Schlauchstück und darin aufgenommener Sonde durch Reibung hindert.

In einer weiteren Ausgestaltung der Erfindung weist das Fußteil eine flächige Fußplatte auf.

Diese Maßnahme hat den erheblichen Vorteil, daß über die Fußplatte ein großflächiger Ansatz auf der Körperfläche möglich ist, so daß die Vorrichtung bestens abgestützt und insbesondere kippsicher auf der Körperoberfläche sitzt.

In einer weiteren Ausgestaltung der Erfindung weist die Hülse einen mittigen Kanal auf, dessen Innendurchmesser dem Außendurchmesser der Sonde entspricht.

Diese Maßnahme hat den Vorteil, daß die Sonde über ihre gesamte Erstreckung in der Hülse exakt ausgerichtet und geführt ist, so daß dann ein innerer exakt runder und ausgerichteter Instrumentenkanal entsteht, obwohl die Sonde an sich aus einem flexiblen Material besteht. Selbst wenn eine solche Sonde aufgrund von unsachgemäßer Lagerung oder durch längeres Belasten einen leicht ovalen oder leicht gequetschten Zustand eingenommen hat, wird diese, wenn sie in diesen Kanal eingeschoben wird, wieder in die exakt runde Geometrie ausgerichtet. Dadurch ist gewährleistet, daß trotz solcher Verformungen in der Vorrichtung ein exakt zylindrischer Instrumentenkanal entsteht.

In einer weiteren Ausgestaltung der Erfindung ist die Hülse proximalseitig über eine Ventilanordnung verschließbar.

Diese Maßnahme hat den Vorteil, daß die Ventilanordnung an einer baulich günstigen und auch für den Operateur von Trokaren her gewohnten Position angeordnet ist, was die Handhabung der Vorrichtung im Sinne einer Trokarhülse erleichtert.

In einer weiteren Ausgestaltung der Erfindung weist die Hülse ein auf das Fußteil dichtend aufsetzbares Kopfteil auf.

Diese Maßnahme hat den Vorteil, daß die Vorrichtung zweiteilig ausgebildet ist, so daß zunächst das Fußteil wie zuvor beschrieben angebracht und mit der Sonde verklemmt werden kann, und daß anschließend dann das Kopfteil dichtend aufsetzbar ist. Dadurch ist es einfach möglich, nach Anbringen des Fußteiles und Verklemmens mit der Sonde diese auf die entsprechende Länge abzulängen, anschließend das Kopfteil, das die eigentliche Hülse trägt, aufzuschieben und dichtend zu verbinden. Auch hier sind durch einfaches Abnehmen Korrekturen möglich, wenn beispielsweise nicht ausreichend abgelängt worden ist.

Auch diese Maßnahme vereinfacht die Handhabung. Auch beim Abnehmen der Vorrichtung nach einem Eingriff ist die Handhabung erleichtert. Es kann nämlich zunächst das Kopfstück abgenommen werden, wobei das Fußteil von dem dann freigelegten Abschnitt der Sonde überragt wird. Dieser Abschnitt kann dann ergriffen werden, um sicherzustellen, daß die Sonde beim Abziehen des Fußteiles nicht versehentlich in den Körper hineingezogen wird.

In einer weiteren Ausgestaltung der Erfindung ist vorgesehen, daß die Hülse ein Innenrohr aufweist, das in die flexible Sonde einschiebbar ist.

Diese Maßnahme hat den Vorteil, daß die flexible Sonde nicht nur von der äußeren Hülse gestützt und versteift wird, sondern noch zusätzlich durch das in diese eingeschobene Innenrohr.

Dieses Innenrohr schützt auch zusätzlich davor, daß bei starkem Klemmdruck der lichte Innenraum der flexiblen Sonde verengt wird.

Dadurch kann nun ein so hoher Klemmdruck ausgeübt werden, daß das Material der flexiblen Sonde zwischen dem steifen Innenrohr und der steifen äußeren Hülse unverrückbar geklemmt ist. Diese Ausgestaltung ist beispielsweise dann von Vorteil, wenn von vornherein erkannt ist, daß der Patient unruhig ist oder regelmäßig hustet, beispielsweise aufgrund eines Raucherhustens.

In einer weiteren Ausgestaltung der Erfindung steht das Innenrohr distalseitig vom Fußteil vor.

Diese Maßnahme hat den Vorteil, daß das Innenrohr nicht nur eine Versteifung der flexiblen Sonde in dem Abschnitt bildet, in dem diese vom Körper absteht, sondern bis beispielsweise in die Bauchdecke hinein.

In einer weiteren Ausgestaltung der Erfindung steht das Innenrohr so weit vor, daß es bis in das körperinnere Hohlorgan reicht.

Diese Maßnahme hat den Vorteil, daß auch Bereiche der Sonde im körperinneren Hohlorgan exakt in Richtung des Innenrohres ausgerichtet sind und somit ein exaktes Einführen von steifen Instrumenten exakt geführt erlauben. Auch hier kann ausgeschlossen werden, daß durch Verlagerung des Patienten die flexible Sonde unmittelbar nach dem Eintritt in das innere Hohlorgan aus der Flucht der Hülse seitlich abgeknickt oder abgeführt wird, was ein Einführen eines Instrumentes in diese abgeknickten Abschnitte erschweren würde.

In einer weiteren Ausgestaltung der Erfindung erstreckt sich das Innenrohr bis zu einem Kopfteil der Vorrichtung hin.

Diese Maßnahme hat den Vorteil, daß vom Kopfteil, also dem proximalen Endbereich der Vorrichtung, in das ein Instrument eingeschoben wird, bis weit in das Innere des Körperhohlorganes ein geradliniger, in seinem Innendurchmesser exakt bestimmter innerer Kanal vorliegt, durch den Instrumente exakt geführt hindurchgeschoben werden können, was die Handhabung wesentlich erleichtert, insbesondere das Einführen in die Sonde.

In einer weiteren Ausgestaltung der Erfindung sind die beweglichen Klemmelemente in Klemmrichtung vorgespannt, und es ist ein Betätigungselement vorgesehen, durch das die Klemmelemente in die nicht-klemmende Stellung bewegbar sind.

Diese Maßnahme hat den Vorteil, daß die Klemmelemente, sowie das Betätigungselement freigegeben wird, in die klemmende Stellung bewegt werden, so daß ein unverlierbarer Sitz der Vorrichtung an der Sonde gewährleistet ist. Soll nun die Vorrichtung weiter längs der Sonde verschoben werden, wird das Betätigungselement betätigt und die Klemmelemente entgegen ihrer Vorspannung bewegt, so daß dann die Hülse längs der Sonde verschoben werden kann.

Dies erleichtert die Handhabung beispielsweise deswegen, daß zunächst einmal die Vorrichtung relativ locker auf der Bauchdecke positioniert angebracht werden kann, nach Freigeben des Betätigungselementes die Klemmelemente aber dann für den zuvor erwähnten unverlierbaren Sitz sorgen. Es können zu diesem Zeitpunkt dann beispielsweise andere Manipulationen, wie z.B. das Ablängen der Sonde, durchgeführt werden. Anschließend kann dann der Relativsitz korrigiert werden, bzw. an der Sonde gezogen werden, um den Magen exakt positioniert an die Innenseite der Bauchdecke heranzulegen.

In einer weiteren Ausgestaltung der Erfindung ist eine Fixiervorrichtung vorgesehen, die die beweglichen Klemmelemente in deren klemmender Stellung fixiert.

Diese Maßnahme hat den Vorteil, daß einerseits die zuvor angegebene einfache Handhabbarkeit ermöglicht ist, andererseits aber durch die Fixiervorrichtung sichergestellt ist, daß die beweglichen Klemmelemente in ihrer klemmenden Stellung fixiert werden können, so daß dann auch durch gewollte oder ungewollte Manipulationen am Betätigungselement kein versehentliches Lösen der Hülse von der Sonde erfolgt.

In einer weiteren Ausgestaltung der Erfindung weist die Fixiervorrichtung einen Fixierring auf, der in einer Drehstellung die beweglichen Klemmelemente fixiert und in einer anderen Drehstellung die Bewegung erlaubt.

Diese Maßnahme hat den Vorteil, daß durch eine einfache Drehbewegung die Fixiervorrichtung entweder in die Fixierstellung oder in die gelöste Stellung gebracht werden kann.

In einer weiteren Ausgestaltung der Erfindung weist das Fußteil zwei relativ zueinander verdrehbare Schalenelemente auf, wobei eines dieser Schalenelemente den Fixierring trägt.

Diese Maßnahme hat den Vorteil, daß die doch relativ großen Schalenelemente eine hervorragende Angriffstelle, beispielsweise für die Hand einer Handhabungsperson, darstellen, um die Drehbewegung der Fixiervorrichtung durchzuführen.

In einer weiteren Ausgestaltung der Erfindung sind die Klemmelemente an auf die Sonde zu beweglichen, von Federn beaufschlagten Bauteilen angeordnet, und das Betätigungselement steht derart mit diesen Bauteilen in Eingriff, daß bei Betätigen dieses Betätigungselements die Klemmelemente gegen die Kraft der Federn von der Sonde weg bewegt werden.

Diese Maßnahme hat den Vorteil, daß die Klemmelemente durch einen einfachen Vorgang, nämlich durch Betätigen des Betätigungselements, bewegt werden können.

In einer weiteren Ausgestaltung der Erfindung ist vorgesehen, daß im Kopfteil zumindest ein Fenster vorhanden ist, über das visuell der Zusammenbau aus Sonde und Kopfteil ersichtlich ist.

Diese Maßnahme hat den Vorteil, daß durch die Fenster der exakte Sitz und der exakte Zusammenbau, insbesondere auch die Ausgestaltung mit dem Innenrohr, visuell inspiziert werden kann.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele im Zusammenhang mit den beiliegenden Zeichnungen näher beschrieben und erläutert. Es zeigen:
- Fig. 1: eine perspektivische, teilweise aufgeschnittene Darstellung einer ersten Ausführungsform der Vorrichtung,
- Fig. 2: eine Explosionsdarstellung, teilweise im Längsschnitt, der Vorrichtung von Fig. 1, wobei ein Kopfteil von einem Fußteil abgehoben ist,
- Fig. 3: einen Zwischenzustand beim Anbringen der Vorrichtung an einem menschlichen Körper, nämlich einen Zustand, nach dem eine Magensonde durch die Magenwand und die Bauchdecke hindurchreichend gesetzt worden ist,
- Fig. 4: eine der Fig. 3 vergleichbare Darstellung, nach der auf den vom Körper abstehenden Endabschnitt der Sonde ein Fußteil der Vorrichtung von Fig. 1 gesetzt worden ist,
- Fig. 5: eine der Darstellung von Fig. 4 vergleichbare Darstellung, nach Bewerkstelligen des Klemmvorganges und nach Ablängen der Sonde,
- Fig. 6: den endfertig montierten Zustand der Vorrichtung, bei dem auf das Fußteil das Kopfteil aufgesetzt worden ist, wobei dargestellt ist, wie ein Instrument in die Vorrichtung eingeschoben werden soll,
- Fig. 7: einen Schnitt längs der Linie VII-VII in Fig. 2 mit eingeschobener Sonde vor Bewerkstelligung der Verklemmung,
- Fig. 8: eine der Fig. 7 entsprechende Darstellung nach Bewerkstelligen der Verklemmung,
- Fig. 9: eine perspektivische Darstellung eines weiteren Ausführungsbeispiels einer erfindungsgemäßen Vorrichtung,
- Fig. 10: eine Explosionsdarstellung der Bauteile der Vorrichtung von Fig. 9,
- Fig. 11: eine Draufsicht auf das in der Explosionsdarstellung von Fig. 10 dargestellte unterste Bauteil mit den beweglichen Klemmelementen,
- Fig. 12: eine der Fig. 11 entsprechende Draufsicht, wobei das in der Explosionsdarstellung von Fig. 10 zweitunterste Bauteil, nämlich der Fixierring, aufgelegt ist,
- Fig. 13: eine der Fig. 12 entsprechende Darstellung, wobei der Fixierring um 90° in Uhrzeigerrichtung verdreht ist,
- Fig. 14: eine der Fig. 12 entsprechende Darstellung, wobei auf diesen Zusammenbau noch das Betätigungselement in Form eines Schiebers aufgelegt ist, also das Bauteil, das in der Explosionsdarstellung von Fig. 10 in der Mitte angeordnet ist, und
- Fig. 15: eine der Fig. 14 entsprechende Darstellung, wobei der Schieber eingedrückt ist, wodurch die Klemmelemente entgegen ihrer Vorspannung in eine nichtklemmende Stellung gebracht worden sind.

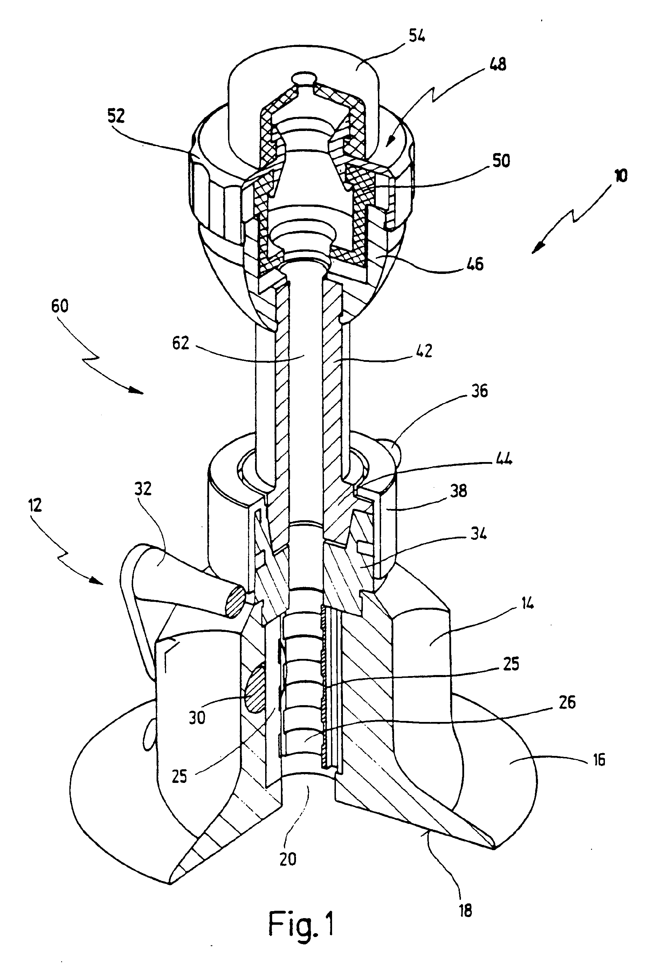

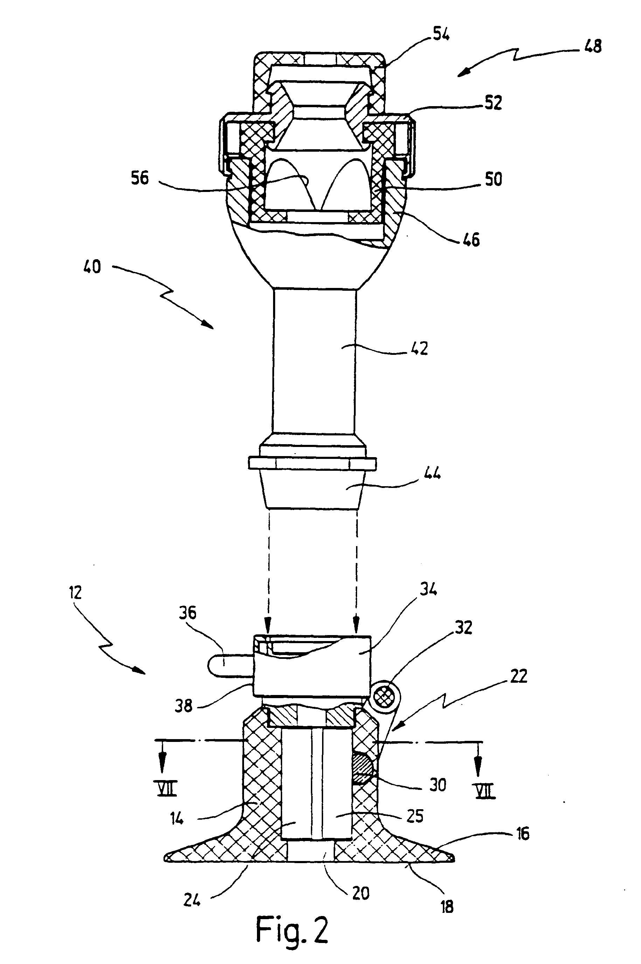

Eine in den Figuren 1 und 2 dargestellte Vorrichtung zum Schaffen eines transkutanen Zuganges zu einem körperinneren Hohlorgan, nämlich zum Magen, ist in ihrer Gesamtheit mit der Bezugsziffer 10 versehen.

Die Vorrichtung 10 weist ein Fußteil 12 auf, wie es inbesondere auch aus der Darstellung von Fig. 2 ersichtlich ist.

Das Fußteil 12 weist einen etwa hohlzylindrischen Körper 14 auf, der in eine durchmessergrößere Fußplatte 16 übergeht.

Über die etwa scheibenförmige Unterseite 18 der Fußplatte 16 wird die Vorrichtung auf einer Körperfläche, beispielsweise der Bauchdecke, eines menschlichen Körpers angesetzt, wie das später noch näher beschrieben wird.

Durch das Fußteil 12 geht eine durchgehende kanalartige Öffnung 20 hindurch. In dem Fußteil 12 ist eine Klemmvorrichtung 22 aufgenommen.

Die Klemmvorrichtung 22 weist zwei Klemmelemente 24 bzw. 25 auf, die die Form von jeweils halben Zylinderschaleh aufweisen. An der Innenseite, also der kanalartigen Öffnung 20 zugewandten Seite, sind die Klemmelemente 24 bzw. 25 mit Vorsprüngen 26 versehen.

Die beiden Klemmelemente 24 und 25 wurden dadurch hergestellt, daß ein mit einem Innengewinde versehenes zylindrisches Rohr der Länge nach in zwei etwa gleiche halbschalenförmige Hälften aufgetrennt worden ist. Das Innengewinde stellt dann die Vorsprünge 26 dar.

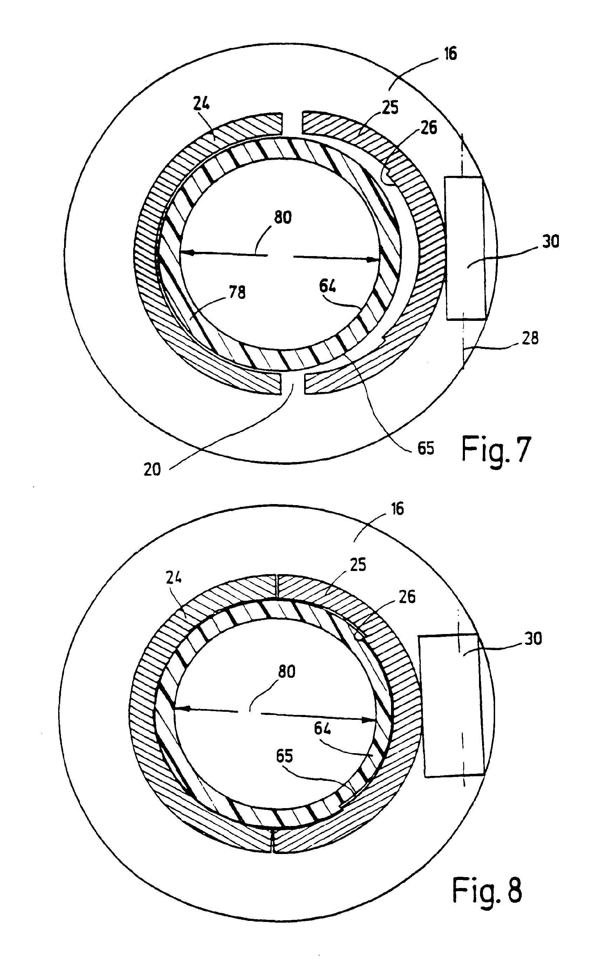

Das Klemmelement 25 steht mit seiner Außenseite mit einem Exzenter 30 einer Exzenterwelle 28 in Berührung, die über einen Hebel 32 verschwenkbar ist. Die Funktionsweise der Klemmvorrichtung 22 wird später im Zusammenhang mit der Funktionsweise der Vorrichtung 10, insbesondere mit den Schnittdarstellungen von den Figuren 7 und 8, näher beschrieben.

Der hohlzylindrische Körper 14 ist an dem der Fußplatte 16 gegenüberliegenden Ende mit einer Kupplung 34 versehen.

Die Kupplung 34 ist als Viergangkupplung ausgebildet und weist einen äußeren Ring 38 auf, der über einen Stift 36 von Hand ergreifbar und drehbar ist.

Die Kupplung 34 dient dazu, um das Fußteil 12 mit einem Kopfteil 40 dichtend zu verkuppeln.

Das Kopfteil 40 weist einen langerstreckten hohlzylindrischen Schaft 42 auf, an dessen in der Darstellung von Fig. 2 unterem Ende ein Kupplungskonus 44 angeordnet ist, der in die Kupplung 34 des Fußteiles 12 paßt.

An dem dem Kupplungskonus 44 gegenüberliegenden Ende ist der Schaft 42 mit einem erweiterten Kopf 46 versehen, der eine Ventilanordnung 48 trägt. Eine Schraubkappe 52 ist auf den Kopf aufdrehbar.

Die Schraubkappe 52 trägt an ihrer Unterseite, die dem Kopf 46 zugewandt ist, ein Lippenventil 50. Ferner ist auf einen mittigen Flansch noch eine endseitige Dichtkappe 54 aus gummielastischem Material aufgesetzt.

Wie insbesondere aus Fig. 2 zu erkennen, ist eine innere Ventilklappe 56 vorhanden, die den Kopf 46 zur Außenseite hin dichtend verschließt. Die Ventilklappe 56 ist jedoch geschlitzt, so daß durch die Kappe 54 hindurch unter Spreizen der Ventilklappe 56 ein Instrument in den Schaft 42 eingeschoben werden kann, wie das nachfolgend noch erläutert wird.

Durch den Zusammenbau von Fußteil 12 und Kopfteil 40, wie er in Fig. 1 dargestellt ist, entsteht eine starre Hülse 60, die einen durchgehenden mittigen Kanal 62 aufweist.

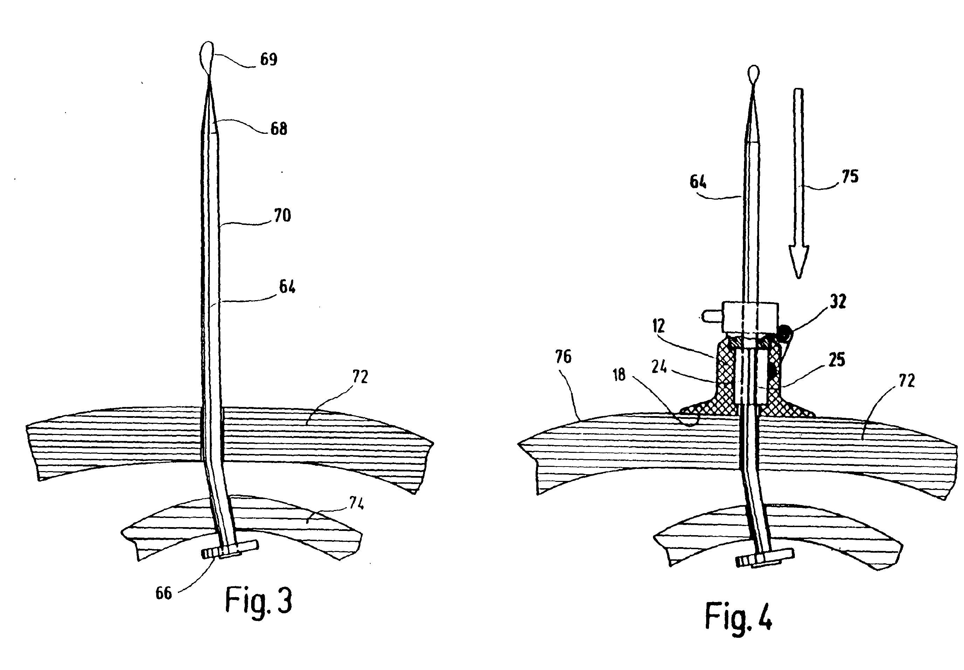

Eine Sonde 64 (siehe Fig. 3) besteht aus einem schlauchförmigen Hohlrohr 70 aus flexiblem Kunststoffmaterial, das an einem Ende mit einer Rückhaltescheibe 66 versehen ist. Am gegenüberliegenden Ende ist ein Konus 68 mit einer Fixierschlaufe 69 vorhanden.

Derartige Sonden sind als Sonden zur intragastralen Langzeiternährung bekannt.

In Fig. 3 ist ein Zustand gezeigt, nach dem die Sonde 64 über die Speiseröhre in den Magen eingeführt und durch die Magenwand 74 und die Bauchdecke 72 zur Außenseite hin geführt ist.

### Dieser Zustand wird wie folgt bewerkstelligt:

Zunächst wird ein Gastroskop, also ein flexibles Endoskop, über die Speiseröhre in den Magen eingeführt, und dieser wird durch Luftinsufflation aufgebläht. Die Spitze des Gastroskopes wird abgewinkelt und an die Stelle an der Innenseite der Magenwand 74 geführt, durch die die Sonde hindurchtreten soll. Von der Außenseite ist dies dadurch zu erkennen, daß diese Stelle durch das Gastroskop von innen beleuchtet wird. Durch entsprechende Abdunklung des Raumes kann diese punkturelle Stelle von außen erkannt werden. Nach großflächig sterilem Abwaschen des vorgesehenen Punktionsbereiches wird eine etwa 4 bis 5 mm breite Stichinzision durchgeführt. Eine entsprechende Punktionskanüle wird unter endoskopischer Kontrolle in den Magen eingeführt, und anschließend wird die Punktionsnadel entfernt. Auf die Kunststoffkanüle wird zunächst eine Einführhilfe für einen Faden aufgesteckt, und der Faden wird in den Magen eingeführt.

Sobald der in den Magen eingeführte Faden durch das Gastroskop ersichtlich ist, wird der Faden im Magen mit einer Biopsiezange erfaßt und mitsamt dem Gastroskop aus dem Körper über die Mundöffnung herausgezogen.

Das proximale Fadenende wird nun mit der Fixierschlaufe 69 der Sonde 64 verknotet.

Anschließend wird die Sonde 64 durch langsamen Zug am distalen Fadenende intragastinal plaziert. Beim Eintritt der Sondenspitze in die noch in der Bauchdecke steckende Kunststoffkanüle ist ein leichter Widerstand spürbar. Die Sonde 64 wird anschließend mit der Kunststoffkanüle durch die Bauchdecke 72 nach außen gezogen, bis die Rückhaltescheibe 66 an der Innenseite der Magenwand 74 anliegt; dieser Zustand ist in Fig. 3 dargestellt.

Die Vorrichtung 10 wird in ihre beiden Einzelteile, nämlich Fußteil 12 und Kopfteil 40, zerlegt.

Über die von der Bauchdecke 72 abstehende Sonde 64 wird nun das Fußteil 12 geschoben, wie das in Fig. 4 durch einen Pfeil 75 angedeutet ist, wobei die Sonde 64 durch die kanalartige, durchgehende Öffnung 20 durchgefädelt wird. Die Klemmvorrichtung 22 befindet sich dabei in ihrer offenen Stellung, d.h. die beiden Klemmelemente 24 und 25 sind voneinander wegbewegt, wie das in der Schnittdarstellung von Fig. 7 dargestellt ist.

Das Fußteil 12 wird so weit aufgeschoben, bis dessen Fußplatte 16 auf der Außenseite 76 der Bauchdecke 72 zum Liegen kommt, wie dies in Fig. 4 dargestellt ist.

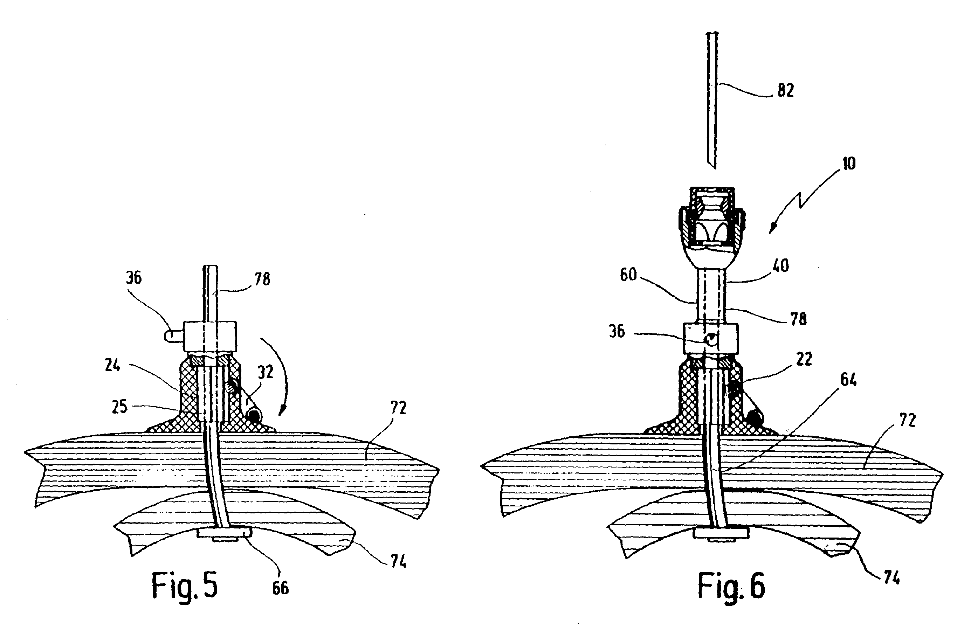

Nunmehr wird die Sonde 64 in dem über das Fußteil 12 hinausragenden Bereich ergriffen, und es wird daran gezogen, bis die Rückhaltescheibe 66 fest an der Innenseite der Magenwand 74 zum Liegen kommt und die Magenwand 74 an die Innenseite der Bauchdecke 72 herangezogen hat, wie das in Fig. 5 ersichtlich ist.

Nunmehr wird durch Umlegen des Hebels 32, wie das in Fig. 5 durch einen Pfeil angedeutet ist, die Klemmvorrichtung 22 betätigt, wodurch das Fußteil 12 mit der Sonde 64 verklemmt wird.

Wie aus dem Übergang von Fig. 7 zu Fig. 8 ersichtlich ist, wird dabei die etwa halbzylinderförmige Schale des Klemmelementes 25 durch den Konus 30 auf das gegenüberliegende Klemmelement 24 bewegt, wodurch ein geschlossener ringförmiger Kanal 62 gebildet wird, in dem passend der vom Körper überstehende Abschnitt 78 der Sonde 64 aufgenommen wird.

Die Vorsprünge 26 an der Innenseite des Klemmelementes 25 graben sich dabei in die Außenseite 65 der Sonde 64 hinein, ohne daß jedoch diese dabei derart verformt wird, daß deren Lumen bzw. deren lichter Innendurchmesser 80 verändert oder beeinträchtigt wird.

Wie insbesondere aus der Schnittdarstellung von Fig. 8 zu entnehmen, sitzt der Abschnitt 78 der Sonde 64 exakt rund ausgerichtet zwischen den Klemmelementen 24 und 25. In diesem geklemmten Zustand entspricht dann der lichte Innendurchmesser des durch die beiden halbschalenförmigen Klemmelemente 24 und 25 gebildeten Kanals 62 dem lichten Außendurchmesser der Sonde 64.

Nach Fixieren der Sonde 64 wird der über die Bauchdecke 72 vorstehende Abschnitt 78 abgelängt, und zwar auf eine solche Länge, daß er sich exakt über die Länge des mittigen Kanals 62 des Schaftes 42 erstreckt.

Das Kopfteil 40 wird in gleicher Richtung wie zuvor das Fußteil 12 über den Abschnitt 78 der Sonde 64 geschoben, bis der Kupplungskonus 44 in die Kupplung 34 des Fußteiles 12 eingetreten ist. Durch Drehen des Ringes 38 über den Stift 36 wird die Kupplung geschlossen, so daß eine dichte Verbindung zwischen Fußteil 12 und Kopfteil 40 geschaffen ist.

Dadurch ist ein von der Fußplatte 16 bis zum Lippenventil 50 durchgehender zylindrischer Kanal 62 geschaffen, dessen lichter Innendurchmesser exakt dem Außendurchmesser des Abschnittes 78 der Sonde 64 entspricht. Das äußere Ende des Abschnittes 78 reicht also gerade bis an das untere Ende des Lippenventiles 50 heran. Dadurch ist eine zur Außenseite hin dichte Verbindung geschaffen. In den Zusammenbau, wie er aus Fig. 6 ersichtlich ist, können nun durch den Kopf 46 Instrumente 82 eingeschoben werden, beispielsweise ein Endoskop oder chirurgische Instrumente, um im Magen chirurgische Eingriffe oder auch lediglich Untersuchungen durchzuführen.

Ist dieser Vorgang beendet, wird die Kupplung 34 wieder gelöst, das Kopfteil 40 abgezogen und die Sonde 64 an dem über das Fußteil 12 überstehenden Abschnitt ergriffen. Anschließend wird durch Umlegen des Hebels 32 die Klemmvorrichtung 22 gelöst, so daß dann das Fußteil 12 so weit angehoben werden kann, daß die Sonde 64 zwischen Unterseite 18 der Fußplatte 16 und der Bauchdecke 72 ergriffen werden kann. Nach Abziehen des Fußteiles 12 kann an den Endabschnitt ein Luer-Lock-Ansatz angebracht werden, über den dann beispielsweise ein Ernährungsbrei dem Magen zugeführt werden kann, die Sonde 64 dann zur intragastralen Langzeiternährung dienen kann.

Soll ein weiterer operativer Eingriff oder eine weitere Inspektion durchgeführt werden, wird der Luer-Lock-Ansatz wieder abgenommen und wie zuvor beschrieben die Vorrichtung 10 angesetzt. Dieser Vorgang kann über Wochen oder Tage gesehen mehrfach durchgeführt werden, wobei lediglich die einzige Inzision, also diejenige, durch die die Sonde 64 hindurchgeführt wurde, notwendig ist. Dies bedeutet für den Patienten eine entscheidend geringere Traumatisierung, da über ein und dieselbe Sonde 64 sowohl die Ernährung als auch operative Eingriffe durchgeführt werden können.

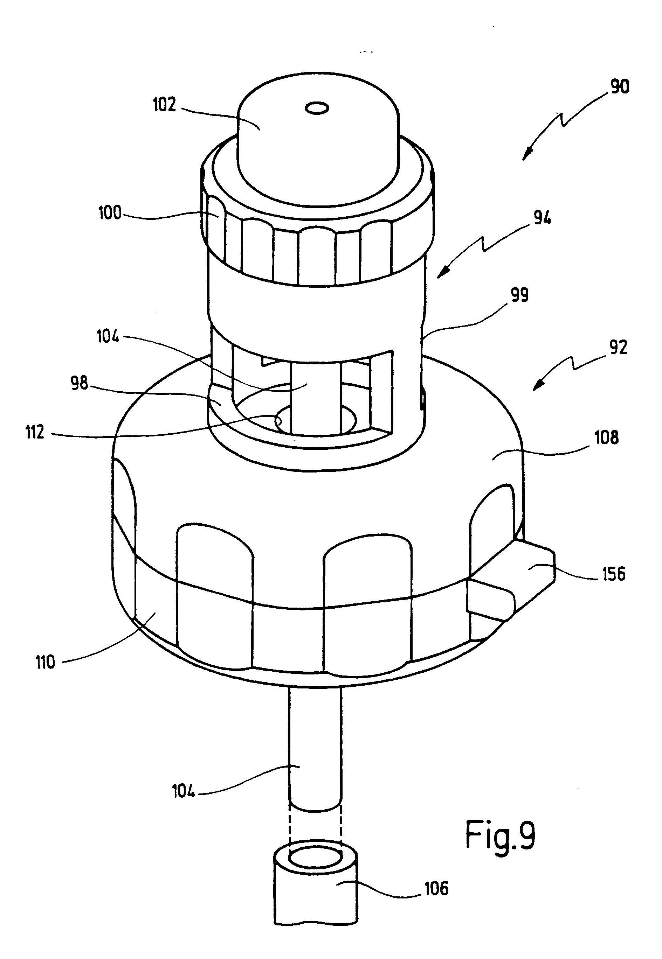

Ein in den Figuren 9 bis 15 dargestelltes weiteres Ausführungsbeispiel einer erfindungsgemäßen Vorrichtung ist in seiner Gesamtheit mit der Bezugsziffer 90 bezeichnet.

Die Vorrichtung 90 weist ein Fußteil 92 und ein Kopfteil 94 auf.

Das Kopfteil 94 weist einen etwa zylindrischen Körper 96 auf, in dem in dessen umfänglicher Wand zwei diametral gegenüberliegende Fenster 98 und 99 ausgespart sind. Am oberen äußeren Ende ist eine Schraubkappe 100 aufgedreht, die proximalseitig mit einer Silikonkappe 102 verschlossen ist.

Im Inneren dieses Bereiches sind, wie zuvor beschrieben, entsprechende Ventilklappen angeordnet, die, falls kein Instrument in die Vorrichtung 90 eingeschoben ist, für einen dichten Abschluß nach proximal sorgen.

Das Kopfteil 94 ist mittig mit einem Innenrohr 104 versehen, das sich bis in den Bereich der Schraubkappe 100 erstreckt.

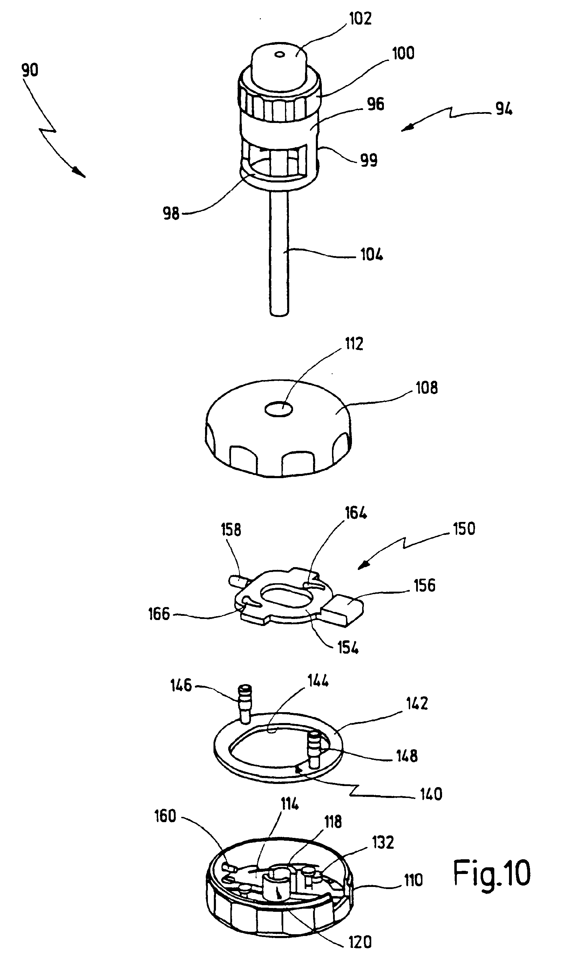

Aus der Explosionsdarstellung von Fig. 10 und der Darstellung des Zusammenbaus von Fig. 9 ist ersichtlich, daß das Innenrohr 104 eine solche Länge aufweist, daß es von der Unterseite des Fußteiles 92 vorsteht.

Der Außendurchmesser des Innenrohrs 104 ist dabei so bemessen, daß dieser in etwa dem lichten Innendurchmesser der Sonde 106 entspricht, in die die Vorrichtung 90 eingeschoben werden soll.

Die Vorrichtung 90 kann so weit eingeschoben, bzw. anders ausgedrückt, die Sonde 106 kann so weit in den Zusammenbau eingeschoben werden, bis die Sonde 106 im Bereich der Sichtfenster 98, 99 zum Liegen kommt, was visuell überprüft werden kann.

Das Fußteil 92 besteht aus zwei Halbschalen 108, 110, die etwa in Form einer Dose zusammengesetzt sind.

Die in der Darstellung von Figuren 9 und 10 obere Halbschale 108 weist eine mittige Öffnung 112 auf, deren Öffnungsquerschnitt so bemessen ist, daß die Sonde 106 hindurch treten kann.

In der unteren Halbschale 110 sind neben weiteren, näher zu beschreibenden Bauelementen noch die beiden in der Explosionsdarstellung ersichtlichen Bauelemente, nämlich eine Fixiervorrichtung 140 und ein Betätigungselement 150, aufgenommen.

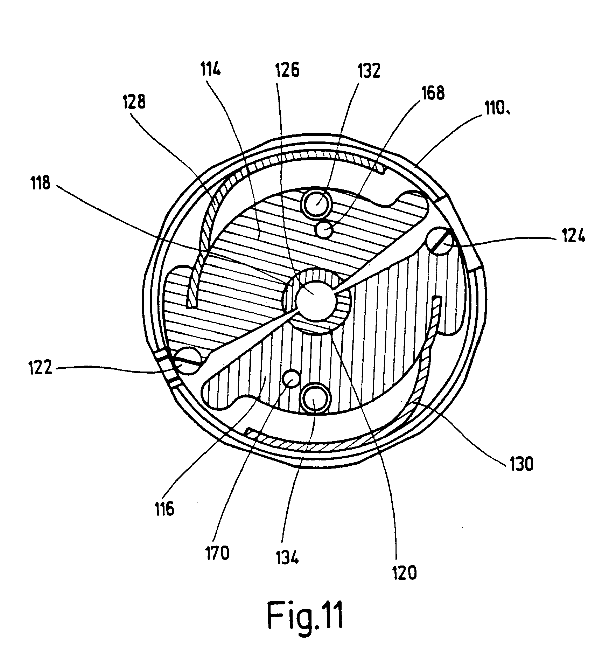

Zunächst sollen aber im Zusammenhang mit Fig. 11 diejenigen Bauteile der unteren Halbschale 110 näher beschrieben werden, die in der Explosionsdarstellung von Fig. 10 nur teilweise ersichtlich sind.

Aus der Draufsicht von Fig. 11 ist zu erkennen, daß im Inneren der Halbschale 110 zwei etwa grob halbkreisförmige plattenförmige Bauteile 114, 116 angeordnet sind, die die etwa jeweils halbzylindrischen, von diesen hochstehenden Klemmelemente 118 bzw. 120 tragen.

Diese beiden Klemmelemente 118, 120 stehen sich vis-à-vis und bilden, wenn sie aneinanderliegen, in etwa einen Hohlzylinder. In dem Hohlzylinder kann dann sowohl die Sonde 106 als auch das Innenrohr 104 Platz finden, dieser bildet also eine Hülse.

Jedes der Bauteile 114 bzw. 116 ist über eine Schraube 122 bzw. 124 verschwenkbar an der Halbschale 110 befestigt.

Jedes der Bauteile 114 bzw. 116 ist über eine Feder 128 bzw. 130 im Gehäuse der Halbschale 110 derart vorgespannt, daß sich die Klemmelemente 118, 120 aufeinander zu bewegen, also in eine klemmende Stellung bewegt werden.

In dieser Stellung umgrenzen diese dann einen inneren Kanal 126.

Jedes der Bauteile 114 bzw. 116 kann gegen die Kraft der jeweiligen Feder 128 bzw. 130 um die Mittellängsachse der jeweiligen Schraube 112 bzw. 124 radial nach außen verschwenkt werden, wobei sich die Klemmelemente 118, 120 von einander weg bewegen, also in die nicht-klemmende Stellung bewegt werden.

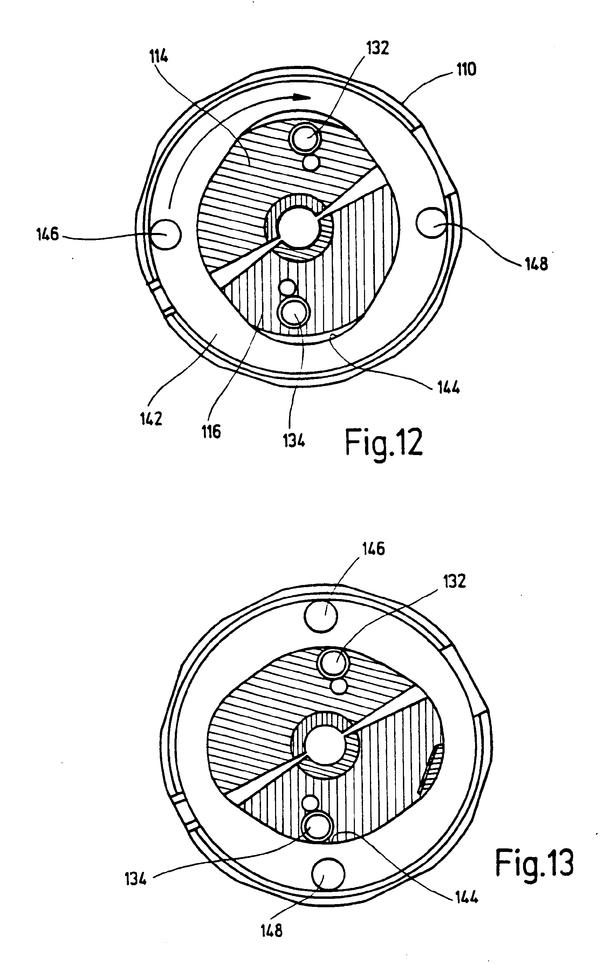

In Fig. 12 ist dargestellt, wie nunmehr auf den Zusammenbau von Fig. 11 die Fixierervorrichtung 140 in Form eines Ringes 142 aufgelegt ist. Es ist ersichtlich, daß der Ring 142 eine nichtkreisförmige innere Kontur 144 aufweist.

Von der Oberseite des Ringes 142 stehen diametral gegenüberliegend zwei Stehbolzen 146 und 148 nach oben vor.

Ebenfalls nach oben steht von den Bauteilen 114 und 116 jeweils ein Sperrbolzen 132 bzw. 134 vor.

Aus der Darstellung von Fig. 12 ist zu erkennen, daß zwischen den Sperrbolzen 132 bzw. 134 und der Kontur 144 des Ringes 142 noch ein gewisser radialer Abstand besteht, d.h. die Bauteile 114, 116 könnten noch gespreizt werden.

Wird der Ring 142 um 90° im Uhrzeigersinn verdreht, wie dies in Fig. 12 durch einen Pfeil dargestellt ist, nimmt der Ring 142 die in Fig. 13 dargestellte Position ein.

In dieser Drehstellung liegt nun der Ring 142 mit seiner Innenkontur 144 sowohl an der radial äußeren Seite des Sperrbolzens 132 als auch an der radial äußeren Seite des Sperrbolzens 134 an. In dieser Drehstellung sperrt also die Fixiervorrichtung 140 davor, daß die Klemmelemente 118, 120 gespreizt bzw. in deren nicht-klemmende Stellung verschoben werden.

Anders ausgedrückt, fixiert und spannt die Fixiervorrichtung 140 die Klemmelemente 118 und 120 in ihrer klemmenden Stellung.

Wie aus Fig. 10 ersichtlich, stehen die beiden Stehbolzen 146 und 148 von der Oberseite des Ringes 142 vor. Diese Stehbolzen 146 bzw. 148 greifen in hier nicht ersichtliche Aussparungen an der Unterseite der oberen Halbschale 108 passend ein. Wird also die Halbschale 108 zur Halbschale 110 verdreht, wird damit automatisch der Ring 142 mit verdreht.

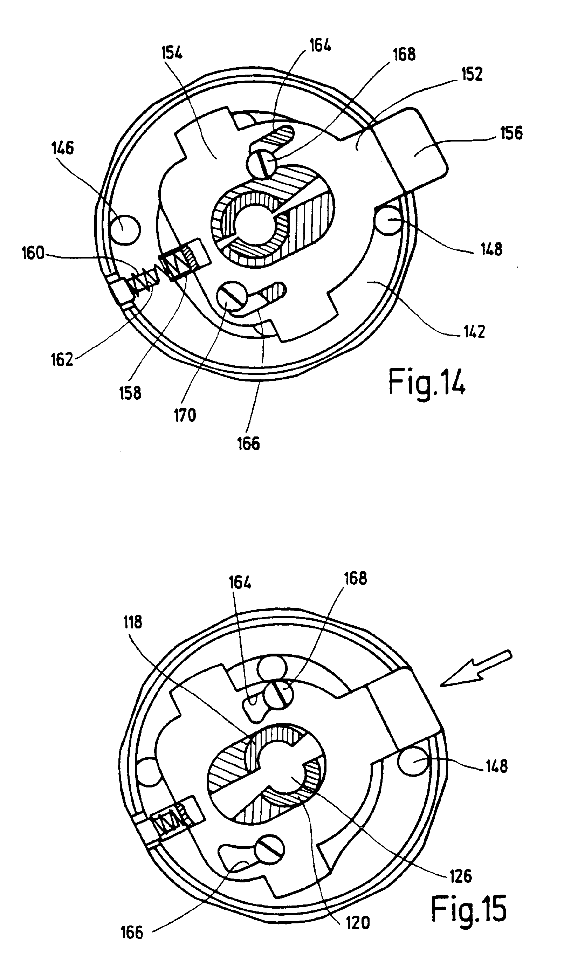

In Fig. 14 ist nun dargestellt, wie auf den Zusammenbau von Fig. 12 noch zusätzlich das Betätigungselement 150 aufgelegt ist.

Das Betätigungselement 150 ist als Schieber 152 ausgebildet und weist in etwa die Form einer Platte 154 mit entsprechenden Aussparungen auf. Eine radial vorstehende Nase 156 steht, bei zusammengebauter Vorrichtung, wie das insbesondere aus Fig. 9 ersichtlich ist, radial von den zusammengebauten Halbschalen 108 und 110 vor. Dies dient als Ansatzpunkt für einen Finger einer Hand, um den Schieber 152 zu betätigen. Diametral gegenüberliegend ist eine Führungshülse 158 vorgesehen (siehe insbesondere Fig. 10), die einen entsprechenden Zapfen 160 an der Innenseite der Halbschale 110 umgreifen kann.

Wie aus der Schnittdarstellung von Fig. 14 zu erkennen, ist in der Führungshülse 158 und auf dem Zapfen 160 eine Feder 162 angeordnet, die die Betätigungsvorrichtung 150 in die in Fig. 14 dargestellte Position schiebt.

In der Platte 150 sind zwei Kulissen 164 und 166 ausgespart, durch die Stifte 168 und 170 ragen, die mit den darunterliegenden Bauteilen 114 bzw. 116 verbunden sind.

Die Kontur der Kulissen 164 bzw. 166 ist nun derart, daß, wenn der Schieber 152 radial nach innen gedrückt ist, wie das in Fig. 15 durch den Pfeil dargestellt ist, die Stifte 168 und 170 radial nach außen gedrückt werden. Dadurch werden die mit diesen verbundenen Bauteile 114 und 116 voneinander weg bewegt und damit die von diesen getragenen Klemmelemente 118 und 120 gespreizt bzw. in die nicht-klemmende Stellung bewegt.

Dies ist insbesondere aus Fig. 15 ersichtlich, d.h. der mittige Kanal 126 hat sich aufgeweitet. In diesem Zustand kann nun ohne Reibschluß die Sonde 106 durchgeschoben werden bzw. über das in dem Zusammenbau in diesem Kanal 126 angeordnete Innenrohr 104 geschoben werden. Die exakte Positionierung der Sonde 106 kann dann über die Fenster 98 bzw. 99 beobachtet werden, und nach aktuellem Sitz braucht lediglich die Nase 156 des Schiebers freigegeben werden, und der Schieber wird wieder von der in Fig. 15 dargestellten Position in die in Fig. 14 dargestellte Position gedrückt. Dies wird zum einen begünstigt durch die Feder 162 und insbesondere durch die beiden Federn 128 und 130.

Beim Anlegen der Vorrichtung 90 an die Sonde 106 kann beispielsweise zunächst der Zusammenbau aus den beiden Halbschalen 108, 109 auf die Sonde 106 aufgefädelt und dann mit der Unterseite der Halbschale 110 auf die Bauchdecke des Patienten gelegt werden. Anschließend kann das Innenrohr samt Kopfteil 94 eingeschoben werden, so weit, bis der entsprechend gewünschte Sitz stattfindet. Dieser Sitz kann noch dadurch korrigiert werden, daß kurzzeitig auf die Nase 156 des Schiebers 152 gedrückt wird und der axiale Sitz korrigiert wird.

## Patentansprüche

1. Vorrichtung zum Schaffen eines transkutanen Zuganges zu einem körperinneren Hohlorgan, insbesondere zum Magen, mit einer hohlrohrförmigen flexiblen Sonde (64, 106), die transkutan in das Hohlorgan legbar ist, mit einer starren Hülse (60), die auf einen vom Körper abstehenden Abschnitt (78) der Sonde (64, 106) derart aufbringbar ist, daß die Hülse (60) diesen Abschnitt (78) umgreift, **dadurch gekennzeichnet, daß** eine Klemmvorrichtung (22) zum lösbaren, jedoch festen Verbinden der Sonde (64, 106) mit der Hülse (60) vorhanden ist, die bewegliche Klemmelemente (24, 25; 118, 120) aufweist, welche zwischen einer nicht-klemmenden und einer klemmenden Stellung bewegbar sind.

2. Vorrichtung nach Anspruch 1, **dadurch gekennzeichnet, daß** ein Fußteil (12, 92) zum Aufsetzen der Vorrichtung (10, 90) auf die Körperfläche vorgesehen ist.

3. Vorrichtung nach Anspruch 1 oder 2, **dadurch gekennzeichnet, daß** eine Ventilanordnung (48) vorgesehen ist, durch die der von der Sonde (64, 106) geschaffene Zugang dicht verschließbar ist.

4. Vorrichtung nach einem der Ansprüche 1 bis 3, **dadurch gekennzeichnet, daß** die beweglichen Klemmelemente (24, 25; 118, 120) an die Außenseite (65) der Sonde (64, 106) anlegbar sind.

5. Vorrichtung nach Anspruch 4, **dadurch gekennzeichnet, daß** die beweglichen Klemmelemente (24, 25; 118, 120) flächig an die Außenseite (65) der Sonde (64, 106) anlegbar sind.

6. Vorrichtung nach Anspruch 4 oder 5, **dadurch gekennzeichnet, daß** die Klemmelemente (24, 25; 118, 120) Vorsprünge (26) aufweisen, die beim Klemmschluß in die flexible Sonde (64, 106) eindringen, ohne jedoch dabei das Lumen (80) der Sonde (64, 106) zu verändern.

7. Vorrichtung nach einem der Ansprüche 2 bis 6, **dadurch gekennzeichnet, daß** das Fußteil (12, 92) als separates Bauteil ausgebildet ist.

8. Vorrichtung nach Anspruch 7, **dadurch gekennzeichnet, daß** das Fußteil (12, 92) die Klemmvorrichtung (22) aufweist.

9. Vorrichtung nach einem der Ansprüche 1 bis 8, **dadurch gekennzeichnet, daß** die Klemmvorrichtung (22) zwei etwa halbschalenförmige Klemmelemente (24, 25; 118, 120) aufweist.

10. Vorrichtung nach einem der Ansprüche 2 bis 9, **dadurch gekennzeichnet, daß** das Fußteil (12, 92) eine durchgehende kanalartige Öffnung (20, 126) aufweist, in der radial beweglich Klemmelemente (24, 25; 118, 120) der Klemmvorrichtung (22) angeordnet sind.

11. Vorrichtung nach Anspruch 1 bis 10, **dadurch gekennzeichnet, daß** die Hülse (60) ein auf das Fußteil (12, 92) dichtend aufsetzbares Kopfteil (40, 94) aufweist.

12. Vorrichtung nach einem der Ansprüche 1 bis 11, **dadurch gekennzeichnet, daß** die Klemmvorrichtung (22) eine über einen äußeren Hebel (32) verdrehbare Exzenterwelle (28) aufweist, deren Exzenter (30) mit zumindest einem Klemmelement (24, 25) der Klemmvorrichtung (22) in Wirkverbindung steht.

13. Vorrichtung nach einem der Ansprüche 1 bis 12, **dadurch gekennzeichnet, daß** die Klemmvorrichtung (22) ein Schlauchstück aufweist, durch das die Sonde (64) hindurchgeführt ist, und daß die beweglichen Klemmelemente (24, 25) der Klemmvorrichtung (22) das Schlauchstück klemmen, wodurch die im Schlauchstück aufgenommene Sonde (64) mit diesem verklemmt ist.

14. Vorrichtung nach einem der Ansprüche 2 bis 13, **dadurch gekennzeichnet, daß** das Fußteil (12) eine flächige Fußplatte (16) aufweist.

15. Vorrichtung nach einem der Ansprüche 1 bis 14, **dadurch gekennzeichnet, daß** die Hülse (60) einen mittigen Kanal (62) aufweist, dessen Innendurchmesser dem Außendurchmesser der Sonde (64) entspricht.

16. Vorrichtung nach einem der Ansprüche 1 bis 15, **dadurch gekennzeichnet, daß** proximalseitig die Hülse (60) über eine Ventilanordnung (48) verschließbar ist.

17. Vorrichtung nach einem der Ansprüche 1 bis 16, **dadurch gekennzeichnet, daß** die Hülse ein Innenrohr (104) aufweist, das in die flexible Sonde (106) einschiebbar ist.

18. Vorrichtung nach Anspruch 17, **dadurch gekennzeichnet, daß** das Innenrohr (104) distalseitig vom Fußteil (92) vorsteht.

19. Vorrichtung nach Anspruch 18, **dadurch gekennzeichnet, daß** das Innenrohr (104) so weit distalseitig vom Fußteil (92) vorsteht, daß es bis in das körperinnere Hohlorgan reicht.

20. Vorrichtung nach einem der Ansprüche 17 bis 19, **dadurch gekennzeichnet, daß** sich das Innenrohr (104) bis zum Kopfteil (94) erstreckt.

21. Vorrichtung nach einem der Ansprüche 1 bis 11, **dadurch gekennzeichnet, daß** die beweglichen Klemmelemente (118, 120) in Klemmrichtung vorgespannt sind, und daß ein Betätigungselement (150) vorgesehen ist, durch das die Klemmelemente (118, 120) in die nicht-klemmende Stellung bewegbar sind.

22. Vorrichtung nach Anspruch 21, **dadurch gekennzeichnet, daß** eine Fixiervorrichtung (140) vorgesehen ist, die die beweglichen Klemmelemente (118, 120) in deren klemmender Stellung fixiert.

23. Vorrichtung nach Anspruch 22, **dadurch gekennzeichnet, daß** die Fixiervorrichtung (140) einen Fixierring (142) aufweist, der in einer Drehstellung die beweglichen Klemmelemente (118, 120) fixiert und in einer anderen Drehstellung deren Bewegung erlaubt.

24. Vorrichtung nach Anspruch 23, **dadurch gekennzeichnet, daß** das Fußteil (92) zwei relativ zueinander verdrehbare Schalenelemente (108, 110) aufweist, wobei eines (110) den Fixierring (142) trägt.

25. Vorrichtung nach einem der Ansprüche 21 bis 24, **dadurch gekennzeichnet, daß** die Klemmelemente (118, 120) an auf die Sonde (106) zu bewegbaren, von Federn (128, 130) beaufschlagten Bauteilen (114, 116) angeordnet sind, und daß das Betätigungselement (150) derart mit diesen Bauteilen (114, 116) in Eingriff steht, daß beim Betätigen des Betätigungselements (150) die Klemmelemente (118, 120) gegen die Kraft der Federn (128, 130) von der Sonde (106) weg bewegt werden.

26. Vorrichtung nach einem der Ansprüche 11 bis 25, **dadurch gekennzeichnet, daß** im Kopfteil (94) zumindest ein Fenster (98, 99) vorhanden ist, über das visuell der Zusammenbau aus Sonde (106) und Kopfteil (94) ersichtlich ist.

## Claims

1. Device for providing transcutaneous access to an internal hollow organ, in particular to the stomach, comprising a tubular flexible probe (64, 106) disposable transcutaneously in the hollow organ and a rigid sleeve (60) attachable to a section (78) of the probe (64, 106) extending from the body, wherein the sleeve (60) encompasses the section (78), **characterized in that** a clamping device (22) is provided for releasably, but fixedly connecting the probe (64, 106) to the sleeve (60), the clamping device having moveable clamping elements (24, 25; 118,120) moveable between a clamping position and a non-clamping position.

2. Device of claim 1, **characterized in that** a base (12, 92) is provided for placing the apparatus (10, 90) on the body surface.

3. Device of claim 1 or 2, **characterized in that** a valve assembly (48) is provided through which the access created by the probe (64, 106) is sealingly closed.

4. Device of anyone of claims 1 through 3, **characterized in that** the moveable clamping elements (24, 25; 118, 120) can be engaged against the outer side (65) of the probe (64, 106).

5. Device of claim 4, **characterized in that** the moveable clamping elements (24, 25; 118, 120) are engageable with the outside (65) of the probe (64, 106) over its surface.

6. Device of claim 4 or 5, **characterized in that** the clamping elements (24, 25; 118, 120) comprise projections (26) which penetrate into the flexible probe (64, 106) when clamped, however without altering the lumen (80) of the probe (64, 106).

7. Device of anyone of claims 2 through 6, **characterized in that** the base (12, 92) is formed as a separate component.

8. Device of claim 7, **characterized in that** the base (12, 92) comprises the clamping device (22).

9. Device of anyone of claims 1 through 8, **characterized in that** the clamping device (22) comprises two approximately half shell-shaped clamping elements (24, 25; 118, 120).

10. Device of anyone of claims 2 through 9, **characterized in that** the base (12, 92) comprises a continuous channel-like opening (20, 126) in which radially moveable clamping elements (24, 25; 118, 120) of the clamping device (22) are arranged.

11. Device of anyone of claims 1 through 10, **characterized in that** the sleeve (60) comprises a headpiece (40, 94) sealingly attachable to the base (12, 92).

12. Device of anyone of claims 1 through 11, **characterized in that** the clamping device (22) comprises a cam shaft (28) rotatable through an outer lever (32), whose cam (30) is in operative connection with at least one clamping element (24, 25) of the clamping device (22).

13. Device of anyone of claims 1 through 12, **characterized in that** the clamping device (22) includes a tube piece through which the probe (64) is passed and **in that** the moveable clamping elements (24, 25) of the clamping device (22) clamp the tube piece, such that the probe (64) within the tube piece is clamped.

14. Device of anyone of claims 2 through 13, **characterized in that** the base (12) comprises a two-dimensional base plate (16).

15. Device of anyone of claims 1 through 14, **characterized in that** the sleeve (60) comprises a central channel (62), whose inner diameter corresponds to the outer diameter of the probe (64).

16. Device of anyone of claims 1 through 15, **characterized in that** the sleeve (60) is closeable via a valve assembly (48) at its proximal side.

17. Device of anyone of claims 1 through 16, **characterized in that** the sleeve comprises an inner tube (104) which can be inserted into the flexible probe (106).

18. Device of claim 17, **characterized in that** the inner tube (104) extends distally from the base (92).

19. Device of claim 18, **characterized in that** the inner tube (104) extends distally from the base (92) to the extent that it can reach into the internal hollow organ.

20. Device of anyone of claims 17 through 19, **characterized in that** the inner tube (104) extends to the headpiece (94).

21. Device of anyone of claims 1 through 11, **characterized in that** the moveable clamping elements (118, 120) are biased in the clamping direction and **in that** an actuator element (150) is provided through which the clamping elements (118 120) are moveable into the non-clamping position.

22. Device of claim 21, **characterized in that** a fixing device (140) is provided which fixes the moveable clamping elements (118, 120) in their clamping position.

23. Device of claim 22, **characterized in that** the fixing device (140) comprises a fixing ring (142), which fixes the moveable clamping elements (118, 120) when said ring is disposed at one angular position and allows their movement when said ring is disposed at another angular position.

24. Device of claim 23, **characterized in that** the base (92) comprises two shell elements (108, 110) relatively rotatable to one another, where one of the shell elements (110) supports the fixing ring (142).

25. Device of anyone of claims 21 through 24, **characterized in that** the clamping elements (118, 120) are arranged on components (114, 116) moveable toward the probe (106) biased by springs (128, 130) and **in that** the actuator element (150) is engaged with these components (114, 116) such that the clamping elements (118, 120) are moved away from the probe (106) against the force of the springs (128, 130) by actuation of the actuator element.

26. Device of anyone of claims 11 through 25, **characterized in that** the headpiece (94) comprises at least one window (98, 99) through which the assembly of the probe (106) with the headpiece (94) can be observed.

## Revendications

1. Dispositif pour pratiquer un accès transcutané à un organe interne creux, en particulier l'estomac, comportant une sonde (64, 106) flexible en forme de tube creux, qui peut être posée par abord transcutané dans l'organe creux, ledit dispositif comportant un manchon (60) rigide, qui est destiné à être posé sur une partie (78) de la sonde (64, 106) en saillie sur le corps, de telle sorte que le manchon (60) entoure cette partie (78), **caractérisé en ce que**, pour l'assemblage amovible, mais ferme de la sonde (64, 106) avec le manchon (60), il est prévu un dispositif de serrage (22) qui comporte des éléments de serrage (24, 25 ; 118, 120) mobiles, qui sont aptes à se déplacer entre une position de non-serrage et une position de serrage.

2. Dispositif selon la revendication 1, **caractérisé en ce qu'**il est prévu un pied (12, 92) pour poser le dispositif (10, 90) sur la surface du corps.

3. Dispositif selon la revendication 1 ou 2, **caractérisé en ce qu'**il est prévu un système de soupape (48), qui permet d'obturer de manière étanche l'accès pratiqué par la sonde (64, 106).

4. Dispositif selon l'une quelconque des revendications 1 à 3, **caractérisé en ce que** les éléments de serrage (24, 25 ; 118, 120) mobiles peuvent être appliqués contre la face extérieure (65) de la sonde (64, 106).

5. Dispositif selon la revendication 4, **caractérisé en ce que** les éléments de serrage (24, 25 ; 118, 120) mobiles peuvent être appliqués à plat contre la face extérieure (65) de la sonde (64, 106).

6. Dispositif selon la revendication 4 ou 5, **caractérisé en ce que** les éléments de serrage (24, 25 ; 118, 120) comportent des saillies (26) qui, au moment de l'assemblage par serrage, s'engagent dans la sonde (64, 106) flexible, sans toutefois modifier la lumière (80) de la sonde (64, 106).

7. Dispositif selon l'une quelconque des revendications 2 à 6, **caractérisé en ce que** le pied (12, 92) est réalisé sous forme de pièce séparée.

8. Dispositif selon la revendication 7, **caractérisé en ce que** le pied (12, 92) comporte le dispositif de serrage (22).

9. Dispositif selon l'une quelconque des revendications 1 à 8, **caractérisé en ce que** le dispositif de serrage (22) comporte deux éléments de serrage (24, 25 ; 118, 120) sensiblement en forme de demi-coque.

10. Dispositif selon l'une quelconque des revendications 2 à 9, **caractérisé en ce que** le pied (12, 92) comporte un orifice traversant (20, 126) en forme de conduit, dans lequel sont agencés les éléments de serrage (24, 25 ; 118, 120) du dispositif de serrage (22), mobiles dans le sens radial.

11. Dispositif selon l'une quelconque des revendications 1 à 10, **caractérisé en ce que** le manchon (60) comporte une partie supérieure (40, 94) apte à être posée de manière étanche sur le pied (12, 92).

12. Dispositif selon l'une quelconque des revendications 1 à 11, **caractérisé en ce que** le dispositif de serrage (22) comporte un arbre d'excentrique (28), qui est apte à être entraîné en rotation par un levier (32) extérieur et dont l'excentrique (30) est en liaison fonctionnelle avec au moins un élément de serrage (24, 25) du dispositif de serrage (22).

13. Dispositif selon l'une quelconque des revendications 1 à 12, **caractérisé en ce que** le dispositif de serrage (22) comporte un tronçon de tuyau flexible, à travers lequel est guidée la sonde (64), et **en ce que** les éléments de serrage (24, 25) mobiles du dispositif de serrage (22) bloquent le tronçon de tuyau, moyennant quoi la sonde (64) logée dans le tronçon de tuyau est bloquée avec celui-ci.

14. Dispositif selon l'une quelconque des revendications 2 à 13, **caractérisé en ce que** le pied (12) comporte une plaque de base (16) plane.

15. Dispositif selon l'une quelconque des revendications 1 à 14, **caractérisé en ce que** le manchon (60) comporte un conduit central (62) dont le diamètre intérieur correspond au diamètre extérieur de la sonde (64).

16. Dispositif selon l'une quelconque des revendications 1 à 15, **caractérisé en ce que** le manchon (60) peut être fermé du côté proximal par un système de soupape (48).

17. Dispositif selon l'une quelconque des revendications 1 à 16, **caractérisé en ce que** le manchon comporte un tube intérieur (104) qui peut être inséré dans la sonde (106) flexible.

18. Dispositif selon la revendication 17, **caractérisé en ce que** le tube intérieur (104) forme, du côté distal, une saillie sur le pied (92).

19. Dispositif selon la revendication 18, **caractérisé en ce que** le tube intérieur (104) forme, du côté distal, une saillie sur le pied (92) sur une distance telle qu'il s'avance jusque dans l'organe creux interne.

20. Dispositif selon l'une quelconque des revendications 17 à 19, **caractérisé en ce que** le tube intérieur (104) s'étend jusqu'à la partie supérieure (94).

21. Dispositif selon l'une quelconque des revendications 1 à 11, **caractérisé en ce que** les éléments de serrage (118, 120) mobiles sont précontraints dans la direction de serrage et **en ce qu'**il est prévu un élément de manoeuvre (150), par lequel les éléments de serrage (118, 120) peuvent être amenés dans la position de non-serrage.

22. Dispositif selon la revendication 21, **caractérisé en ce qu'**il est prévu un dispositif d'immobilisation (140) par lequel les éléments de serrage (118, 120) mobiles sont immobilisés dans leur position de serrage.

23. Dispositif selon la revendication 22, **caractérisé en ce que** le dispositif d'immobilisation (140) comporte une bague d'immobilisation (142) qui, dans une position de rotation, immobilise les éléments de serrage (118, 120) mobiles et, dans une autre position de rotation, permet leur mouvement.

24. Dispositif selon la revendication 23, **caractérisé en ce que** le pied (92) comporte deux coques (108, 110) pouvant tourner l'une par rapport à l'autre, l'une d'entre elles (110) portant la bague d'immobilisation (142).

25. Dispositif selon l'une quelconque des revendications 21 à 24, **caractérisé en ce que** les éléments de serrage (118, 120) sont agencés sur des éléments (114, 116) pouvant être déplacés sur la sonde (106) et sollicités par des ressorts (128, 130), et **en ce que** l'élément de manoeuvre (150) est en prise avec les éléments (114, 116) de telle sorte que lors de l'actionnement de l'élément de manoeuvre (150), les éléments de serrage (118, 120) sont déplacés en s'écartant de la sonde (106) à l'encontre de la force des ressorts (128, 130).

26. Dispositif selon l'une quelconque des revendications 11 à 25, **caractérisé en ce qu'**il est prévu dans la partie supérieure (94) au moins une fenêtre (98, 99), par l'intermédiaire de laquelle il est possible de voir l'ensemble formé par la sonde (106) et la partie supérieure (94).
